# EUROPEAN PATENT SPECIFICATION

(11) **EP 2 403 564 B1**
(45) Date of publication and mention of the grant of the patent: **25.04.2018**
(21) Application number: 10706225.9
(22) Date of filing: 01.03.2010
(51) Int. Cl.: A61M 5/31

(54) **MEDICATION DELIVERY DEVICE WITH FOLDABLE FINGER PAD**
ARZNEIMITTELABGABEVORRICHTUNG MIT ZURÜCKKLAPPBAREN FINGERAUFLAGEN
Dispositif médical d'administration avec éléments d'appui escamotables pour doigts

(30) Priority: 02.03.2009 EP 09002922
(43) Date of publication of application: 11.01.2012
(73) Proprietor: Sanofi-Aventis Deutschland GmbH, 65929 Frankfurt am Main (DE)
(72) Inventor: LANIN, Irina, 65926 Frankfurt am Main (DE); FORYS, Bernhard, 65926 Frankfurt (DE); CLARKE, Alastair, Holmes Chapel Cheshire Crewe CW48BE (GB); EKMAN, Matthew, Macclesfield Cheshire SK10 1RD (GB); GOODE, Kirsten, 65929 FRANKFURT AM MAIN (DE); HEALD, Michael, Maidenhead Berkshire SL6 7SG (GB); HILES, John, Holmes Chapel Cheshire Crewe CW48BE (GB); SMITH, Chris, Holmes Chapel Cheshire CW47QG (GB); HAINS-GADD, Lilly, Queens Close OXFORD OX2A 4HN (GB)
(74) Representative: Weilnau, Carsten
(86) International application number: PCT/EP2010/052529
(87) International publication number: WO 2010/100104

(56) References cited:
- WO-A-92/06725
- FR-A- 2 866 573
- US-A- 2 461 481
- US-A- 2 829 643
- US-A- 3 384 082
- US-A- 5 347 913
- US-A- 5 715 554
- US-A- 5 886 839
- US-A- 5 925 032
- US-A1- 2004 136 062

## Description

Medication delivery device and method of preparing a medication delivery device The invention concerns a medication delivery device and a method of preparing a medication delivery device.

A medication delivery device may have finger pads which can be engaged with the fingers or which prevent sliding movement of fingers gripping the device.

A medication delivery may be designed as pen-type medication delivery device which is suitable to deliver e.g. human growth hormone, heparin or insulin.

A medication delivery device may be designed as a syringe having a plunger that fits tightly in a barrel. The plunger can be pulled and pushed along inside the barrel, allowing the syringe to take in and expel a liquid or gas through an orifice, e.g. a needle, at the distal end of the barrel.

A syringe may have a flange located at the distal end of the barrel, the flange serving as finger pad. The flange can be engaged with two fingers so that the thumb of the user can press the plunger.

If the flange is relatively small, the syringe may be difficult to handle, in particular, if the user has large hands or movement disorders.

A syringe may be supplied with a separate accessory that the user can attach to the syringe in order to provide the user with improved handling of the syringe. Such an accessory requires a manual assembly step by the user which is inconvenient for the user and may be difficult to accomplish, in particular, if the user has large hands or movement disorders. FR 2866573 shows a hinged collar which is attached to a syringe. US 5925032 shows a syringe cannula holder which also comprises a hinged collar. The collars may serve as finger pads. WO 92/06725 shows foldable stabilising tabs which do not serve as finger pads. US 2461481 shows a syringe having a metal barrel which is made of a curved metal plate. During manufacturing tongues cut in the metal plate are turned outwards and longitudinally embossed so that they form stiff handgrips. US3384082 and US2829643 show a medication delivery device according the the preamble of claim 1. For this purpose a medication delivery device is provided. The medication delivery device is configured to deliver medication when a button element is moved. The medication delivery device has a folding finger pad.

If the medication delivery device is not used, the folding finger pad is in a hinged position. This is beneficial for transportation and storage purposes, in particular if the medication must be stored in refrigerated conditions. In use the medication delivery device facilitates comfortable handling when the finger pad is swung out.

Medication delivery devices include auto-injectors, pen-type delivery devices and syringes. The finger pad is suitable to be engaged with the fingers of a user so that the user can hold the device, e.g. during use of a syringe in order to expel the medication from the syringe. Another embodiment of a finger pad is suitable to prevent sliding movement of fingers which grip the device, e.g. during use of a pen-type delivery device.

One embodiment of the medication delivery device is designed as a syringe. A syringe having folding finger pads can be manufactured in the same production line as a conventional syringe. A part of the medication delivery device which comprises the finger pad is built in the medication delivery device instead of a conventional part or additionally to the conventional parts during the manufacturing process. The finger pad is moveable from a first position to a second position. The finger pad is positioned in the first position when the medication delivery device is not used, e.g. during transportation or when the medication delivery device is provided. The finger pad is positioned in the second position when the medication delivery device is used so that the medication is expelled. Preferably, the syringe has two folding finger pads.

In one embodiment of the medication delivery device the angle between the finger pad and an outside wall of the medication delivery device is larger in the second position than in the first position. The outside wall of the medication delivery device is the part of the medication delivery device which is located adjacent to the finger pad in the first position. The outside wall may be the outside wall of the part of the medication delivery device to which the finger pad is coupled. In one embodiment the finger pad is connected with a collar which is located at the proximal end of a barrel of a syringe. In an alternative embodiment the finger pad is connected with a collar which is located at the proximal end of a housing of a pen-type medication delivery device. The angle between the finger pad and an outside wall of the collar is larger in the second position than in the first position. The finger pad has a bottom side and a top side. In the first position the bottom side is adjacent to an outside wall of the medication delivery device. In other words, the finger pad extends along the outside wall of the medication delivery The finger pad extends parallel or nearly parallel with respect to the outside wall. In one embodiment the outside comprises a cavity in which the finger pad is at least partly located in the first position.

In one embodiment the finger pad extends angular with respect to the outside wall of the medication delivery device if the finger pad is positioned in the second position. The finger pad which is positioned in the second position can be engaged with the fingers of the user or prevents sliding movement of the fingers when the device is used. Advantageously, the finger pad extends rectangular or nearly rectangular with respect to the outside wall of the medication delivery device.

The finger pad is configured so that a force which is directed to the proximal direction can impact to the finger pad so that the finger pad remains in the second position when a second force which is directed to the distal direction is impacting to the button element thereby moving the button element to the distal direction. In other words, the finger pad remains in the second position when the medication delivery device is used so that the finger pad can be engaged with the fingers or prevents sliding movement of fingers gripping the device. The medication delivery device comprises hinge means which are configured to couple the finger pad with the outside wall of the medication delivery device. The hinge means facilitate the movement of the folding finger pad so that it can be moved from the first position to the second position. The hinge means is designed as an integral hinge which means that the hinge and the parts to be connected are made of one piece. The medication delivery device comprises stopping means which are configured to stop the movement of the finger pad when the finger pad has reached the second position. The stopping means prevents further movement of the finger pad, which may cause damage of the hinge. One embodiment of the stopping means is designed as protrusion. One embodiment of stopping means is designed as latching means. One embodiment of stopping means is designed as engaging means. One embodiment of the medication delivery device is pre-filled, which means that the medication delivery device contains a medicament when the medication delivery device is provided. One embodiment is a pre-filled disposable syringe, which means the syringe is configure as single-useable syringe.

A method of preparing a medication delivery device which has a folding finger pad comprises moving the finger pad from a first position to a second position before the medication delivery device is used.

In one embodiment the medication delivery device has an outside wall. Preferably, the method comprises the movement of the finger pad so that the angle between the finger pad and the outside wall of the medication delivery device is increased when the finger pad is moved from the first position to the second position.

The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane such as hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCI or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Other features will become apparent from the following detailed description when considered in conjunction with the accompanying drawings.
Figure 1 shows an embodiment of a medication delivery device.
Figure 2 shows an embodiment of a syringe.
Figure 3 shows an embodiment of a collar of the syringe.
Figure 4 shows an integral hinge in detail.
Figure 5 shows a further embodiment of a collar of the syringe.

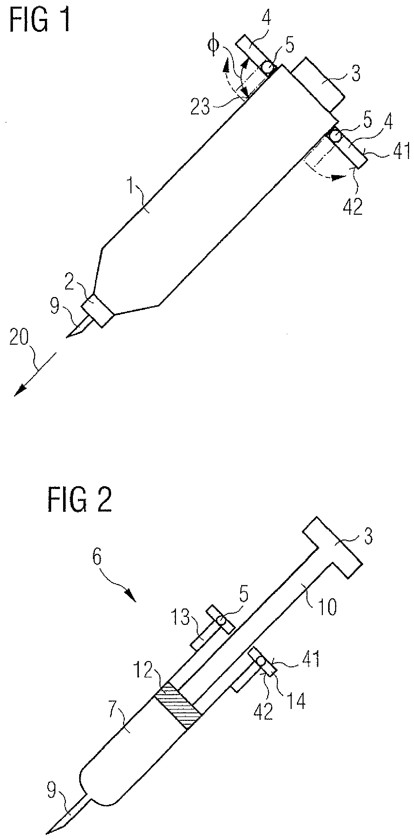

Figure 1 shows an embodiment of a medication delivery device having a body 1, which is suitable to contain medication. A needle unit 2 with a needle 9 is located at the distal end of the body 1. A button element 3 is located at the proximal end of the body 1. The medication delivery device is configured to deliver a dose of the medication through the needle 9 of the needle unit 2 when the button element 3 is moved. In one embodiment the medication is expelled when the button element 3 is pushed into the distal direction with respect to the body 1.

The medication delivery device has folding finger pads 4 which are coupled with the body 1 via hinges 5. The finger pads 4 have top sides 41 and bottom sides 42. The embodiment shown in figure 1 has two folding finger pads 4 which are arranged on opposite sides at the proximal part of the body 1. In alternative embodiments (not shown) more or less folding finger pads are provided.

The finger pads 4 are moveable from a first position to a second position. The first position of the finger pads 4 is shown as a dotted line in figure 1. In the first position the finger pads 4 are positioned so that they extend in the direction of the longitudinal axis 20 of the medication delivery device. In one embodiment the finger pads extends parallel or nearly parallel with respect to the barrel 7. The bottom side 42 of the finger pads is located adjacent to the outside wall 23 of the body 1. In one embodiment the bottom side 42 or regions of the bottom side 42 abut on the outside wall 23 of the body 1. In one embodiment (not shown) the outside wall comprises a cavity, wherein at least a part of the finger pad is positioned in the cavity of the outside wall if the finger pad is positioned in the first position.

When the finger pad 4 is moving from the first position to the second position, the finger pad 4 rotates around the hinge 5. The second position of the finger pads 4 is shown as a straight line in figure 1. In the second position the finger pad 4 extends angular with respect to the outside wall 23 of the medication delivery device. Preferably, the angle between the finger pad 4 which is positioned in the second position and the outside wall 23 of the body 1 is rectangular or nearly rectangular.

The angle between the finger tab 4 and the outside wall 23 of the body 1 in the second position is larger than the angle between the finger tab 4 and the outside wall 23 of the body 1 in the first position.

The finger pads 4 are positioned in the first position when the medication delivery device is not used, e.g. during transportation.

The finger pads 4 are moved to the second position before use of the medication delivery device. In an alternative embodiment (not shown) the finger pads 4 will move automatically from the first position to or towards the second position when the medication delivery device is prepared for use, e.g. upon removal from its external packaging. This can be accomplished by incorporating spring features that urge the finger pads 4 from the first to the second position. Such spring features can be either plastic or metal and may be a separate component or may be an integrated feature of the finger flaps 4, e.g. the spring feature is provided by the geometry of the hinges 5.

In one embodiment of the medication delivery device the medication is delivered when the index, middle, ring and little fingers grip the body 1 of the medication delivery device, the thumb pushing the button element 3 to the distal direction with respect to the body 1.

The finger pads 4 in the second position serve as stopping means for preventing sliding movement of the fingers gripping the body 1. The sliding movement of the fingers is stopped when the finger which is positioned near the finger pads 4, e.g. the index finger, reaches the finger pads 4.

One embodiment of the medication delivery device is an auto-injector configured to deliver a given dose of the medication. In one embodiment the dose is fixed. In an alternative embodiment the dose is adjustable by a user. An alternative medication delivery device is a pen-type medication delivery device.

Figure 2 shows an embodiment of a syringe 6 comprising a plunger 10 that fits tightly in a tube shaped barrel 7 of the syringe 6.

The plunger 10 comprises a button element 3 which is located at the proximal end of the plunger 10. A plunger seal 12 is located at the distal end of the plunger 10. The plunger 10 is moveable with respect to the barrel 7, wherein the plunger seal 12 is moveable inside the barrel 7 along the longitudinal axis of the barrel 7.

One embodiment of the barrel 7 is integrally constructed of glass or plastic. A needle 9 is located at the distal end of the barrel 7, wherein the medication is taken in or expelled through the needle 9. A collar 13 with folding finger pads 14 is located at the proximal end of the barrel 7. The collar 13 guides the moving plunger 10.

The finger pads 14 are formed to be engaged with fingers of a user. One embodiment of a finger pad 14 is formed as a plate. One embodiment of the finger pad (not shown) is rounded so that a recessed grip is formed. Each finger pad 14 is coupled to the collar 13 via a hinge 5. In one embodiment the collar 13 and the barrel 7 are made of one piece, e.g. both are made of plastic. In an alternative embodiment the collar 13 and the barrel 7 are made of different pieces, e.g. the collar 13 is made of plastic and the barrel 7 is made of glass. In one embodiment (not shown) the syringe does not comprise a collar, the finger pads being coupled to another part of the syringe, e.g. the barrel 7.

The finger pads 4 are movable from a first position to a second position. In the first position (not shown in figure 2) the finger pads 4 are positioned so that they extend along the axial direction of the syringe 6. In one embodiment the finger pads are positioned parallel or nearly parallel with respect to the outside wall of the barrel 7 and/or the collar 13. In one embodiment the bottom side 42 of the finger pads abut on the outside wall of the barrel 7.

When the finger pads 4 are moved from the first position to the second position, the finger pads 14 rotate around the hinge 5. In the second position the finger pads 4 extend angular with respect to the axial direction 20 so that the finger pads 5 can be engaged by two fingers of the index, middle, ring and little fingers, e.g. the index finger and the middle finger, so that the user's thumb can press the button element 3 in the distal direction. Preferably, the angle between the finger pads 4 and the outside wall of the barrel 7 is rectangular or nearly rectangular.

In one embodiment the hinge 5 is configured to stop the rotational movement of the finger pads 4. In another embodiment stopping means are provided which are configured to stop the rotational movement when the finger pads reach the second position. Embodiments of stopping means are described below.

When the syringe is used to take in the medication, the plunger 10 is moved in the proximal direction with respect to the barrel 7 so that the plunger seal 12 is moved from the distal end of the barrel 7 in the proximal direction. This movement facilitates taking a liquid into the syringe 2 when the top of the needle 9 is placed in the liquid.

An alternative embodiment of the syringe is pre-filled, which means that the barrel 7 already contains the medication if the syringe 2 is provided. Pre-filled syringes may be disposable.

The medication is expelled through the needle 9 when the plunger 10 is moved to the distal direction with respect to the barrel 7.

The finger pads 4 are moved to the second position before the medication is used to expel the medication. In one embodiment the finger pads 4, which are in the second position, are engaged by the index finger and the middle finger when the thumb presses the button element 3 of the plunger into the distal direction with respect to the barrel 7.

In an alternative embodiment (not shown) the finger pads 14 will move automatically from the first position to or towards the second position when the syringe is prepared for use, e.g. upon removal from its external packaging, priming of the device, etc. This can be accomplished by incorporating one or more spring features that urge the finger pads 14 from the first to the second position upon activation (e.g. upon removal from the external packaging of the device or upon deactivation of one or more stop members that hinder the spring feature from moving the finger pads from the first to the second position). Such spring feature can be made from any suitable material, such as plastic or metal. The spring feature may be designed as one or more separate components or may be an integrated feature of the finger flaps 14, e.g. the spring feature is provided by the geometry of the hinges 5.

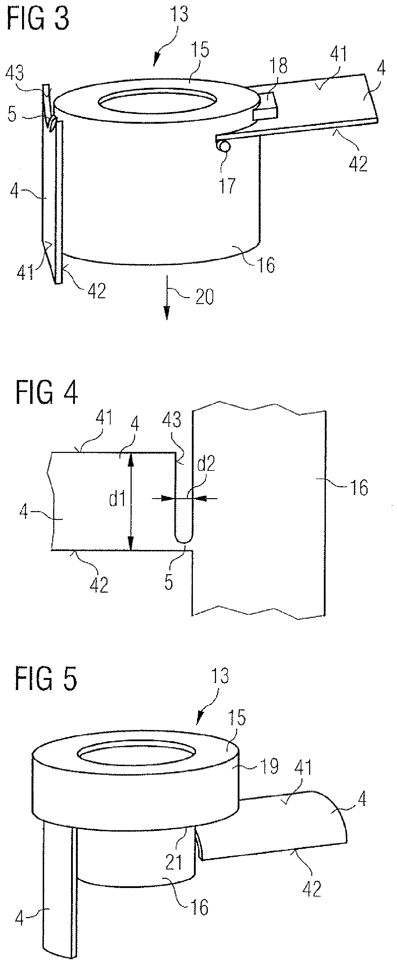

Figure 3 shows an embodiment of the collar 13 with finger pads 4 in more detail.

The collar 13 has a top part 16 which covers the proximal edge of the barrel 7 (not shown in figure 3). A hole is located in the top part 15 through which the plunger 10 (not shown in figure 3) can be moved. A side part 16 of the collar surrounds the side walls of the proximal end of the barrel 7 (not shown in figure 3).

The collar 13 comprises two folding finger pads 4. They are located opposite each other. Each finger pad 4 comprises a top side 41, a bottom side 42 and a lateral side 43 which is adjacent to a hinge 5 for coupling the finger pad 4 to the side part 16 of the collar. Each finger pad 4 is coupled with the side part 16 of the collar via an integral hinge 5, which means that the finger pad 4, the collar 13 and the hinge 5 are made of one piece. One embodiment of the integral hinge 5 is formed as a thin-walled connection. An alternative hinge (not shown) comprises more than one thin-walled connection, e.g. two thin-walled connections which are arranged next to another.

Figure 4 shows the integral hinge 5 in detail. The thin-walled connection forming the hinge 5 connects the bottom part of the lateral side 43 of the finger pad 4 with the side part 16 of the collar. In an alternative embodiment (not shown) the thin-walled connection forming the hinge 5 connects the bottom side 42 of the finger pad 4 which is located near the lateral side 43 with the side part 16 of the collar. In one embodiment the hinge 5 does not extend across the entire with of the lateral side 43, the hinge 5 merely joins a central range of the bottom part of the lateral side 43, as shown in figure 3. In one embodiment (not shown) the hinge 5 extends across the entire or nearly the entire width of the lateral side 43. In one embodiment (not shown) the hinge comprises one or more thin-walled connections which do not have to be located at the central range of the lateral side 43.

When the finger pad 4 is moved to the second position, the rotational movement is stopped when the lateral side 43 reaches the side part 16 of the collar. The thickness d1 of the finger pad 4 is preferably larger than length d2 of the thin-walled connection between the lateral side 43 and the side part 16 of the collar.

The finger pads 4 are moveable from a first position to a second position. In the first position the bottom side 42 of the finger pad is located adjacent to the side part 16 of the finger flange. The finger pad 4 shown on the left hand side of figure 3 is positioned in the first position. In the second position the finger pad 4 extends nearly rectangular with respect to the side part 16 of the collar. The finger pad 4 shown on the right hand side of figure 3 is positioned in the second position.

A lateral side 43 of the finger pad which is located adjacent to the hinge 5 is shaped as sector of a circle so that the form of the lateral side 43 and the form of the side part 16 of the collar fit together if the finger pad 4 is positioned in the second position. In one embodiment (not shown) the lateral side which is located adjacent to the hinge 5 is straight.

In one embodiment (not shown) the thin-walled connection is deposited to the top of the lateral side 43 or to a region between the top and the bottom of the lateral side 43. This embodiment may have stopping means configured to stop the rotational movement of the finger pad, when the finger pad is positioned rectangular or nearly rectangular with respect of the side part 16 of the collar.

Figure 3 shows stopping means 17 which are provided to stop the rotational movement of the finger pad 4 when the finger pad 4 has reached the second position. For clarity reasons the stopping means 17 are shown only on the right hand side of figure 3. The stopping means are designed as bumps 17 located on the outside wall of the side part 16 of the collar. The bumps are positioned so that the region of the finger pads 4 which is located near the outer area of the lateral side 43 abuts on the bumps when the finger pad 4 has been moved to the second position.

An alternative embodiment of stopping means is designed as extruding part 18 of the side part 16 of the collar, the extruding part being arranged so that the top side 41 of the finger pad is moved towards the extruding part 18 when the finger pad reaches the second position.

Figure 5 shows an embodiment of a collar 13, wherein a circumferential flange 19 forms the extruding part serving as stopping means. The circumferential flange 19 stops the rotational movement of the finger pad 4, when the top side 41 of the finger pad reaches the bottom 21 of the flange (shown on the right hand side).

Another difference between the embodiment shown in figure 3 and the embodiment shown in figure 5 is that the finger pads 4 are rounded so that the bottom side of the finger pads 4 abuts on the side part 16 of the collar if the finger pad 4 is positioned in the first position (shown on the left hand side).

It should be mentioned that in one embodiment (not shown) the finger pads are connected to the barrel or another part of the syringe.

### Reference numerals

- 1: body
- 2: needle unit
- 3: button element
- 4: finger pad
- 5: hinge
- 6: syringe
- 7: barrel
- 9: needle
- 10: plunger
- 12: plunger seal
- 13: collar
- 15: top part of collar
- 16: side part of collar
- 18: stopping means
- 19: flange
- 20: longitudinal axis
- 21: bottom of flange
- 23: outside wall
- 41: top side of finger pad
- 42: bottom side of finger pad
- 43: lateral side of finger pad
- d1: thickness
- d2: distance
- φ: angle

## Claims

1. Medication delivery device being configured to deliver medication when a button element (3) is moved, the medication delivery device having:
- a collar (13) with a side part (16) and configured to be located at a proximal end of a barrel (7) of a syringe, and
- a folding finger pad (4) movable from a first position to a second position
wherein the finger pad (4) has a bottom side (42) and a top side (41), the bottom side (42) extending parallel or nearly parallel with respect to an outside wall (23) of the collar (13) when the finger pad (4) is in the first position,
- wherein in the second position the finger pad (4) extends angularly with respect to the outside wall (23), **characterized in that**
- the finger pad (4) is coupled with the side part (16) of the collar (13) by an integral hinge (5), wherein a thin- walled connection forming the hinge (5) connects a bottom part of a lateral side (43) of the finger pad (4) with the side part (16) of the collar (13) such when the finger pad (4) is moved to the second position, the rotational movement is stopped when the lateral side (43) reaches the side part (16) of the collar (13).

2. Medication delivery device according to claim 1 being designed as a syringe (6).

3. Medication delivery device according to claim 1 or 2, wherein in the second position the finger pad (4) extends rectangular or nearly rectangular with respect to the outside wall (23) of the medication delivery device.

4. Medication delivery device according to any one of the preceding claims, wherein the finger pad (4) is configured so that a force which is directed to the proximal direction can impact to the finger pad (4) so that the finger pad remains in the second position when a second force which is directed to the distal direction is impacting to the button element (3) thereby moving the button element (3) to the distal direction.

5. Medication delivery device according to any one of the preceding claims, wherein the hinge means is spring-loaded.

6. Medication delivery device according to any of the preceding claims, being pre-filled with a medicament.

7. Medication delivery device according to any of the claims 1 to 6, having at least two folding finger pads (4).

8. Method of preparing a medication delivery device according to any one of the preceding claims for use comprising the movement of the finger pad (4) from the first position to the second position.

9. Method according to claim 8, wherein the movement of the finger pad (4) from the first to the second position is driven by a spring mechanism.

## Patentansprüche

1. Medikamenten-Verabreichungsvorrichtung, die dazu ausgebildet ist, ein Medikament zu verabreichen, wenn ein Tastenelement (3) bewegt wird, wobei die Medikamenten-Verabreichungsvorrichtung Folgendes aufweist:
- einen Bund (13) mit einem Seitenteil (16), wobei der Bund dazu ausgestaltet ist, an einem proximalen Ende eines Zylinders (7) einer Spritze angeordnet zu sein, und
- eine klappbare Fingerleiste (4), die aus einer ersten Position in eine zweite Position bewegbar ist, wobei die Fingerleiste (4) eine Unterseite (42) und eine Oberseite (41) hat, wobei sich die Unterseite (42) parallel oder nahezu parallel zu einer Außenwand (23) des Bunds (13) erstreckt, wenn die Fingerleiste (4) in der ersten Position ist,
- wobei sich die Fingerleiste (4) in der zweiten Position winkelförmig zu der Außenwand (23) der Medikamenten-Verabreichungsvorrichtung erstreckt, **dadurch gekennzeichnet, dass**
- die Fingerleiste (4) über ein integrales Scharnier (5) mit dem Seitenteil (16) des Bunds (13) gekoppelt ist, wobei eine das Scharnier (5) bildende dünnwandige Verbindung einen Unterteil einer lateralen Seite (43) der Fingerleiste (4) mit dem Seitenteil (16) des Bunds (13) verbindet, so dass, wenn die Fingerleiste (4) in die zweite Position bewegt wird, die Drehbewegung gestoppt wird, wenn die laterale Seite (43) das Seitenteil (16) des Bunds (13) erreicht.

2. Medikamenten-Verabreichungsvorrichtung nach Anspruch 1, welche als eine Spritze (6) ausgebildet ist.

3. Medikamenten-Verabreichungsvorrichtung nach Anspruch 1 oder 2, wobei sich die Fingerleiste (4) in der zweiten Position rechtwinklig oder nahezu rechtwinklig zu der Außenwand (23) der Medikamenten-Verabreichungsvorrichtung erstreckt.

4. Medikamenten-Verabreichungsvorrichtung nach einem der vorhergehenden Ansprüche, wobei die Fingerleiste (4) so konfiguriert ist, dass eine in die proximale Richtung gerichtete Kraft auf die Fingerleiste (4) einwirken kann, so dass die Fingerleiste in der zweiten Position bleibt, wenn eine in die distale Richtung gerichtete zweite Kraft auf das Tastenelement (3) einwirkt, wodurch das Tastenelement (3) in die distale Richtung bewegt wird.

5. Medikamenten-Verabreichungsvorrichtung nach einem der vorhergehenden Ansprüche, wobei das Scharniermittel federgelagert ist.

6. Medikamenten-Verabreichungsvorrichtung nach einem der vorhergehenden Ansprüche, die mit einem Medikament vorgefüllt ist.

7. Medikamenten-Verabreichungsvorrichtung nach einem der Ansprüche 1 bis 6, die mindestens zwei klappbare Fingerleisten (4) hat.

8. Verfahren zur Vorbereitung einer Medikamenten-Verabreichungsvorrichtung nach einem der vorhergehenden Ansprüche, umfassend das Bewegen der Fingerleiste (4) aus der ersten Position in die zweite Position.

9. Verfahren nach Anspruch 8, wobei die Bewegung der Fingerleiste (4) aus der ersten in die zweite Position durch einen Federmechanismus angetrieben wird.

## Revendications

1. Dispositif d'administration de médicament configuré pour administrer un médicament lorsqu'un élément formant bouton (3) est déplacé, le dispositif d'administration de médicament comportant :
- une bague (13) comportant une partie latérale (16) et configurée pour être placée au niveau d'une extrémité proximale d'un cylindre (7) d'une seringue, et
- un appui pour doigt (4) pliable pouvant être déplacé d'une première position à une seconde position, l'appui pour doigt (4) comportant un côté inférieur (42) et un côté supérieur (41), le côté inférieur (42) s'étendant parallèlement ou presque parallèlement par rapport à une paroi extérieure (23) de la bague (13) lorsque l'appui pour doigt (4) se trouve dans la première position,
- l'appui pour doigt (4) s'étendant, dans la seconde position, de manière oblique par rapport à la paroi extérieure (23) du dispositif d'administration de médicament, **caractérisé en ce que**
- l'appui pour doigt (4) est accouplé avec la partie latérale (16) de la bague (13) par le biais d'une charnière intégrée (5), un raccordement à paroi mince formant la charnière (5) raccordant une partie inférieure d'un côté latéral (43) de l'appui pour doigt (4) avec la partie latérale (16) de la bague (13) de telle sorte que, lorsque l'appui pour doigt (4) est déplacé jusqu'à la seconde position, le mouvement de rotation est arrêté lorsque le côté latéral (43) atteint la partie latérale (16) de la bague (13).

2. Dispositif d'administration de médicament selon la revendication 1, étant conçu sous la forme d'une seringue (6).

3. Dispositif d'administration de médicament selon la revendication 1 ou 2, dans lequel l'appui pour doigt (4), dans la seconde position, s'étend à angle droit ou presque à angle droit par rapport à la paroi extérieure (23) du dispositif d'administration de médicament.

4. Dispositif d'administration de médicament selon l'une quelconque des revendications précédentes, dans lequel l'appui pour doigt (4) est configuré de telle sorte qu'une force qui est dirigée dans la direction proximale puisse agir sur l'appui pour doigt (4) de telle sorte que l'appui pour doigt reste dans la seconde position lorsqu'une seconde force qui est dirigée dans la direction distale agit sur l'élément formant bouton (3) de façon à déplacer l'élément formant bouton (3) dans la direction distale.

5. Dispositif d'administration de médicament selon l'une quelconque des revendications précédentes, dans lequel le moyen formant charnière est sollicité par ressort.

6. Dispositif d'administration de médicament selon l'une quelconque des revendications précédentes, celui-ci étant pré-rempli avec un médicament.

7. Dispositif d'administration de médicament selon l'une quelconque des revendications 1 à 6, comportant au moins deux appuis pour doigt (4) pliables.

8. Procédé de préparation d'un dispositif d'administration de médicament selon l'une quelconque des revendications précédentes pour son utilisation comprenant le déplacement de l'appui pour doigt (4) de la première position à la seconde position.

9. Procédé selon la revendication 8, dans lequel le déplacement de l'appui pour doigt (4) de la première à la seconde position est mis en oeuvre par un mécanisme à effet de ressort.
